# FASCICULE DE BREVET EUROPEEN

(11) **EP 0 686 964 B1**
(45) Date de publication et mention de la délivrance du brevet: **15.04.1998**
(21) Numéro de dépôt: 95401359.5
(22) Date de dépôt: 12.06.1995
(51) Int. Cl.: G10K 15/02, A61B 5/12

(54) **Procédé et système pour produire un signal analogique de synthèse**
Verfahren und Anordnung zum Erregen eines analogen synthetisierten Signals
Method and arrangement to generate an analog synthesized signal

(30) Priorité: 10.06.1994 FR 9407084
(43) Date de publication de la demande: 13.12.1995
(73) Titulaire: ETAT FRANCAIS Représenté par le Délégué Général pour l'Armement, 75007 Paris 7ème (FR)
(72) Inventeur: Boisrayon, Michel, F-83330 Le Beausset (FR); Briolle, Françoise, F-13008 Marseille (FR)

(56) Documents cités:
- GB-A- 2 126 443
- US-A- 4 466 325
- US-A- 4 476 724

## Description

L'invention se situe dans le domaine de la synthèse de signaux analogiques à partir de signaux numériques.

Les signaux artificiels ou synthétiques sont couramment utilisés notamment pour produire des sons qui se propagent dans l'air ou dans l'eau. Dans le domaine de l'acoustique aérienne, les synthétiseurs et les orgues électroniques sont bien connus et ont fait l'objet de développements industriels importants car ils touchent un large public. En acoustique sous-marine, les signaux artificiels sont employés pour recréer un environnement virtuel et forme les personnels navigants à identifier des situations opérationnelles complexes. Dans la plupart des cas, on utilise des signaux numériques échantillonnés où chaque échantillon est un nombre représentatif de l'amplitude du signal et qui conserve une même valeur durant la période d'échantillonnage.

On connaît ainsi le brevet GB 2 126 443 qui décrit un procédé pour produire un signal analogique sinusoïdal de synthèse à partir d'un signal numérique généré par un compteur consistant à, utiliser le bit de poids le plus fort comme entrée d'un multiplexeur analogique apte à transformer le signal d'entrée en un signal analogique et les autres bits pour commander un atténuateur apte à moduler le signal analogique.

Actuellement, les signaux numériques sont habituellement calculés au moyen d'un ordinateur par des méthodes très variées. Ils sont ensuite transformés en grandeur analogique, c'est-à-dire en une grandeur variant continûment en fonction du temps. Cette grandeur peut être par exemple un signal électrique, magnétique ou une pression acoustique. Cette transformation est assurée par des appareils appelés convertisseurs numérique-analogique.

Les signaux sont calculés par l'ordinateur, échantillon par échantillon, pour représenter leur évolution temporelle, avec une précision plus ou moins grande. Ces échantillons sont transmis, par exemple par l'intermédiaire d'un bus, au convertisseur numérique-analogique qui convertit les échantillons numériques en grandeurs analogiques pour fournir en sortie le signal analogique.

La précision de restitution du signal analogique dépend des caractéristiques du convertisseur numérique-analogique. L'une de ces caractéristiques est le nombre d'échelons de qualification, c'est-à-dire le nombre de niveaux discrets dont on dispose pour représenter le signal analogique. Ce nombre conditionne la résolution du convertisseur.

Chaque niveau discret est généralement exprimé par un nombre binaire composé d'un nombre déterminé de bits. Ainsi, l'imprécision sur la valeur d'un échantillon correspond à la valeur représentée par le bit de poids faible du nombre binaire représentant l'échantillon. Cette imprécision, habituellement appelée bruits de quantification, conduit à définir un rapport signal-à-bruit directement lié au nombre de bits utilisés pour représenter l'échantillon. Ainsi, lorsque l'amplitude du signal synthétisé est importante, toute la dynamique du convertisseur numérique-analogique est utilisée, donc le rapport signal-à-bruit est élevé. Par contre, des signaux de faible amplitude sont représentés avec moins de précision, le codage utilisant moins de bits. En conséquence, le rapport signal-à-bruit diminue et la distorsion augmente.

La technique utilisée actuellement pour augmenter ce rapport signal-à-bruit consiste à utiliser des convertisseurs numérique-analogique de plus grande résolution, donc ayant un plus grand nombre de bits, dans les limites autorisées par la technologie. Cette solution présente cependant l'inconvénient de nécessiter des convertisseurs à haute résolution pour pouvoir synthétiser des signaux ayant une dynamique importante.

Aussi, l'invention a pour but de remédier à cet inconvénient en proposant un procédé de synthèse de signaux analogiques avec une résolution optimale tout en utilisant des convertisseurs à plus faible résolution.

Dans ce but, l'invention a pour objet un procédé pour produire un signal analogique de synthèse à partir d'un signal numérique consistant :
- à mettre ledit signal numérique sous la forme d'un premier signal numérique de forme d'onde représenté par au plus un nombre prédéterminé de bits supérieur à 1 et d'un second signal numérique d'amplitude,
- à convertir ledit premier signal numérique en un troisième signal analogique au moyen d'un convertisseur numérique-analogique dimensionné pour convertir des nombres binaires comportant un nombre de bits au plus égal audit nombre prédéterminé et
- à moduler ledit troisième signal analogique par ledit second signal numérique au moyen d'au moins un atténuateur programmable recevant ledit troisième signal analogique sur son entrée analogique et ledit second signal numérique sur son entrée numérique de programmation d'atténuation.

Avantageusement, l'atténuateur sera dimensionné pour permettre une atténuation d'au moins 10 décibels, avec une précision d'au moins 5 centièmes de décibel.

L'invention a également pour objet un système de synthèse pour la mise en oeuvre du procédé précédemment défini. Le système est caractérisé en ce qu'il comporte :
- une unité de calcul pour élaborer lesdits premier et second signaux numériques,
- un convertisseur numérique-analogique,
- une première liaison parallèle reliant ladite unité à l'entrée dudit convertisseur numérique-analogique pour transmettre ledit premier signal numérique,
- une seconde liaison parallèle reliant ladite unité à l'entrée numérique de programmation dudit atténuateur pour transmettre ledit second signal numérique
et en ce que la sortie du convertisseur numérique-analogique est reliée à l'entrée analogique de l'atténuateur.

Le procédé et le système de synthèse selon l'invention trouvent une application particulièrement intéressante dans le domaine des tests auditifs ou psycho-acoustiques. Dans ces applications, le signal de synthèse sert à activer un transducteur électroacoustique, du type casque ou analogue, porté par l'utilisateur subissant le test.

D'autres aspects et avantages de l'invention apparaîtront dans la suite de la description en référence aux figures.
- La figure 1 illustre un procédé de synthèse de signaux selon l'état de la technique.
- La figure 2 est un schéma de principe d'un convertisseur numérique-analogique.
- La figure 3 représente les variations en fonction du temps de l'amplitude d'un signal synthétisé.
- La figure 4 schématise le procédé de synthèse selon l'invention.
- La figure 5 représente une variante du procédé selon l'invention.
- La figure 6 représente un système de synthèse selon l'invention, appliqué à des tests psycho-acoustiques.

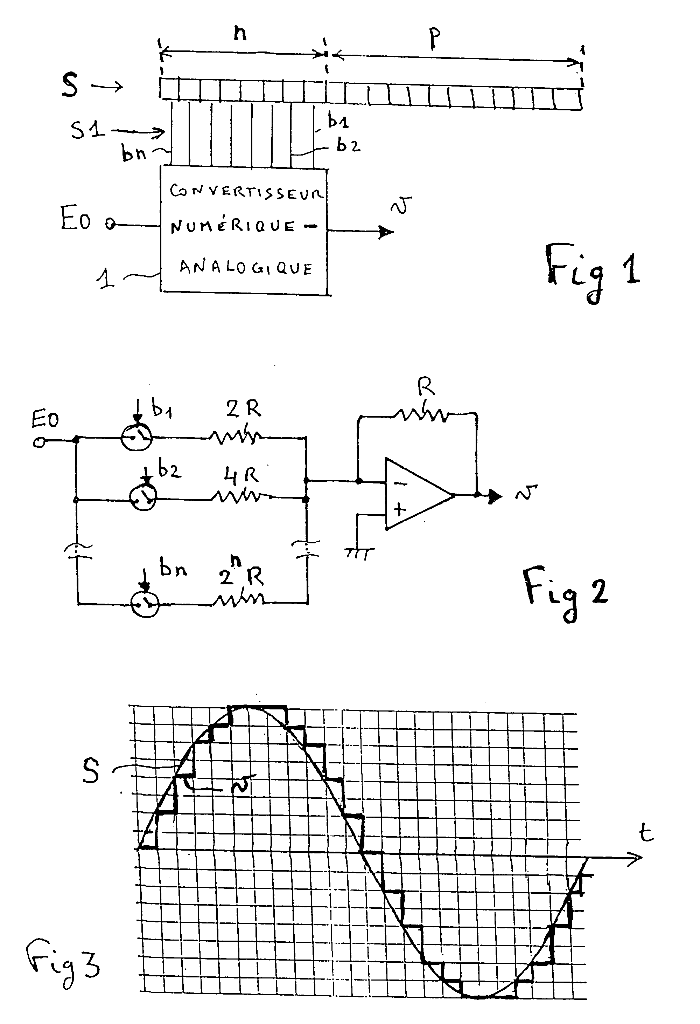

Comme représenté sur la figure 1, un signal à synthétiser se présente généralement sous la forme d'un signal numérique binaire S constitué d'un nombre de bits déterminé. Ce nombre est imposé par le choix de l'unité de calcul qui élabore le signal numérique S.

Ainsi, avec les micro-ordinateurs actuellement disponibles, le signal S peut être défini sur 8, 16 ou 32 bits.

La solution idéale pour produire un signal analogique de synthèse à partir d'un tel signal numérique serait donc d'utiliser un convertisseur numérique-analogique capable de convertir des nombres binaires comportant un nombre de bits au moins égal à celui qui constitue le signal numérique. Toutefois, pour des raisons de coût ou de limitation imposée par la technologie, on est généralement amené à utiliser des convertisseurs ayant une résolution inférieure. On aboutit donc à la solution représentée à la figure 1 où seuls les n bits de poids fort b1, b2, ..., bn du signal numérique disponible sont appliqués à l'entrée du convertisseur 1. Il en résulte que les p bits de poids faible du signal S ne sont pas pris en compte dans la conversion.

La figure 2 montre un schéma de principe classique des convertisseurs numérique-analogique habituellement utilisés. On peut facilement vérifier que le signal analogique v qu'il fournit est une tension proportionnelle au signal numérique S1 formé des bits de poids fort b1-bn. Le signal v est également proportionnel à une tension de réglage E0. Des convertisseurs fondés sur le principe du schéma de la figure 2 sont proposés par divers fabricants. En pratique, la réalisation prévoit bien entendu des moyens de filtrage des harmoniques d'échantillonnage ainsi que la possibilité de fournir des tensions négatives.

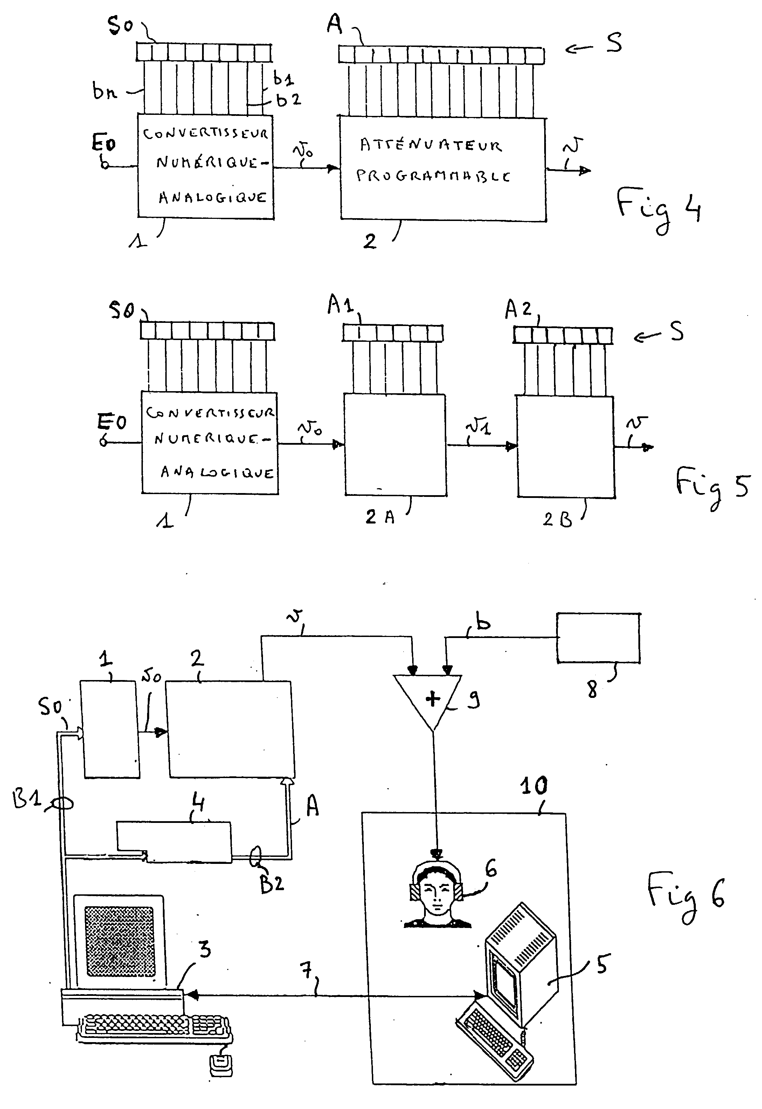

A titre d'illustration, la figure 3 montre les variations en fonction du temps d'un signal v obtenu après conversion d'un signal numérique sinusoïdal S. Le signal v qui n'est défini que pour des valeurs discrètes est donc entaché d'un bruit de quantification permettant de définir un rapport signal-à-bruit caractérisant la conversion effectuée. On considère en général que le rapport signal-à-bruit est égal à autant de fois 6 décibels qu'il y a de bits utilisés pour la conversion.

Ainsi, la méthode qui vient d'être présentée ne permet pas de restituer avec précision les faibles amplitudes des signaux à grande dynamique. Cette solution ne sera donc pas facilement utilisable pour réaliser par exemple des tests psycho-acoustiques où le rapport des amplitudes, exprimé en décibel, des signaux de forte et faible amplitudes peut être de l'ordre de 120 décibels. Bien que la synthèse numérique permette de maîtriser parfaitement les caractéristiques des signaux (caractéristiques spectrales, précision d'amplitude) la résolution des convertisseurs numérique-analogique n'est pas suffisante pour restituer de cette façon des signaux de faible amplitude avec une précision convenable. Comme la dynamique du système auditif est plus élevée que celle des convertisseurs couramment disponibles, on rencontre également ce type de problème dans toutes les applications impliquant des signaux sonores comme la synthèse des signaux musicaux ou la simulation d'environnement dans les sous-marins.

La figure 4 représente de façon schématique le procédé de synthèse conforme à l'invention. Le signal numérique S à synthétiser est mis sous la forme d'un signal de forme d'onde S0 associé à un signal d'atténuation A. Le signal S0 est appliqué à l'entrée d'un convertisseur numérique-analogique 1 qui fournit le signal analogique v0 de forme d'onde correspondant. Le signal v0 est ensuite appliqué à l'entrée analogique d'un atténuateur programmable 2 dont l'entrée numérique de programmation reçoit le signal numérique d'atténuation A. L'atténuateur 2 fournit en sortie le signal analogique de synthèse v.

Le convertisseur 1 fonctionne toujours à résolution constante. Le signal S0 étant défini sur un nombre de bits réduit mais suffisant pour représenter la forme du signal, il est possible d'utiliser des convertisseurs courants peu onéreux. Comme d'autre part, un atténuateur programmable est un appareil travaillant sur des signaux analogiques, sa précision et sa plage de réglage par la commande numérique A ne sont pas soumises aux limitations d'un convertisseur numérique-analogique. Il en résulte que le procédé permet de synthétiser des signaux de très grande dynamique avec une résolution optimale.

L'élaboration des signaux de forme d'onde S0 et d'atténuation A est avantageusement effectuée par une unité de calcul, telle qu'un micro-ordinateur, programmée en fonction de l'application envisagée. Les valeurs d'atténuation A peuvent être préprogrammées, par exemple dans le cas d'une série d'essais prévus à l'avance, soit imposées par l'opérateur à partir du clavier du micro-ordinateur.

La figure 5 représente schématiquement une variante du procédé selon l'invention. Selon cette variante, l'atténuation s'effectue au moyen de plusieurs atténuateurs 2A, 2B montés en cascade et commandés respectivement par des signaux numériques d'atténuation A1, A2. Cette réalisation modulaire permet d'étendre à volonté les plages de variation de l'atténuation en utilisant des circuits standards.

La figure 6 montre un exemple de réalisation détaillée d'un système permettant la mise en oeuvre du procédé selon l'invention. Cette réalisation est présentée dans le cadre d'une application à des tests psycho-acoustiques.

L'unité de calcul 3 est un micro-ordinateur muni d'un microprocesseur travaillant sur des mots d'au moins 8 bits. Le micro-ordinateur est relié par un bus B1 au convertisseur numérique-analogique 1. La sortie v0 du convertisseur 1 est reliée à l'entrée analogique de l'atténuateur programmable 2 qui fournit en sortie le signal de synthèse v. Le micro-ordinateur 3 est d'autre part relié à l'entrée numérique de programmation de l'atténuateur 2 par l'intermédiaire d'une carte d'interface parallèle 4 et d'un second bus B2. Les bus B1, B2 constituent deux liaisons parallèles servant à véhiculer respectivement les signaux de forme d'onde S0 et d'atténuation A. La carte d'interface parallèle 4 a pour but de maintenir constante la valeur de l'atténuation A. En pratique, la carte 4 et le convertisseur 1 sont enfichés dans le fond de panier du micro-ordinateur et reçoivent leurs informations par un bus conforme au standard prévu pour le micro-ordinateur.

Pour effectuer les tests psycho-acoustiques, le système est complété par un sommateur 9 dont une première entrée reçoit le signal de synthèse v et un signal dit "masquant", constitué d'un bruit à large bande de fréquence, d'amplitude fixe fourni par un générateur de bruit blanc 8. La sortie du sommateur 9 alimente un transducteur électro-acoustique du type casque d'écoute. Le casque 6 est porté par l'auditeur dans un local insonorisé 10 dans lequel est placé un terminal 5 relié au micro-ordinateur 3 par une liaison série 7.

Pour effectuer une expérience de psycho-acoustique visant à mesurer les performances de filtrage du système auditif, on mélange le signal masquant b et un signal de synthèse v de forme sinusoïdale dont l'amplitude et la fréquence varient au cours de l'expérience. L'amplitude doit être extrêmement précise (tolérance typique de 0,1 décibel) et considérablement variable (80 décibels). La fréquence de ce signal est également contrôlée avec précision. Les signaux v et b combinés dans le sommateur 9 sont présentés à l'auditeur qui doit indiquer s'il détecte le signal sinusoïdal. Selon sa réponse, on doit modifier l'amplitude du signal. Cette modification peut être effectuée par l'utilisateur au moyen du terminal 5. Ce terminal pourra également être utilisé pour saisir les réponses de l'utilisateur et les transmettre au micro-ordinateur 3 par la liaison 7. L'expérience peut être ainsi entièrement gérée par ordinateur.

A titre d'exemple non limitatif, nous allons enfin donner quelques références de matériels utilisables pour constituer le système de la figure 6. L'ordinateur 3 est le modèle Mackintosh IIx de la société APPLE, qui est relié au convertisseur 1 par une liaison NuBus. Le convertisseur 1 est le modèle NB-A2100 de la société NATIONAL INSTRUMENTS dont la pleine résolution (16 bits) peut être utilisée. L'atténuateur programmable 2 est commercialisé par la société WILSONICS PATT et l'interface parallèle 4 est le modèle NB-DIO 32 de NATIONAL INSTRUMENTS.

## Revendications

1. Procédé pour produire un signal analogique de synthèse (v) à partir d'un signal numérique (S) consistant :
- à mettre ledit signal numérique (S) sous la forme d'un premier signal numérique de forme d'onde (SO) représenté par au plus un nombre prédéterminé (n) de bits (b1, b2, bn) supérieur à 1 et d'un second signal numérique d'amplitude (A),
- à convertir ledit premier signal numérique (SO) en un troisième signal analogique (vO) au moyen d'un convertisseur numérique-analogique (1) dimensionné pour convertir des nombres binaires comportant un nombre de bits au plus égal audit nombre prédéterminé (n) et
- à moduler ledit troisième signal analogique (vO) par ledit second signal numérique (A) au moyen d'au moins un atténuateur programmable (2, 2A, 2B) recevant ledit troisième signal analogique et ledit second signal numérique (A) sur son entrée numérique de programmation d'atténuation.

2. Procédé selon la revendication 1, caractérisé en ce que lesdits premier et second signaux numériques (SO, A) sont produits par une unité de calcul (3) munie d'au moins un micro-processeur travaillant sur des mots d'au moins 8 bits.

3. Procédé selon l'une des revendications 1 ou 2, caractérisé en ce que ledit atténuateur programmable (2, 2A, 2B) est dimensionné pour permettre une atténuation d'au moins 10 décibels.

4. Procédé selon l'une des revendications 1 à 3, caractérisé en ce que ledit atténuateur programmable (2, 2A, 2B) est prévu pour atteindre une précision meilleure que 5 centièmes de décibels.

5. Système de synthèse d'un signal analogique (v) pour la mise en oeuvre du procédé selon l'une des revendications 1 à 4, caractérisé en ce qu'il comporte:
- une unité de calcul (3) pour élaborer lesdits premier et second signaux numériques (SO, A),
- un convertisseur numérique - analogique (1),
- une première liaison parallèle (B1) reliant ladite unité (3) à l'entrée dudit convertisseur numérique-analogique (1) pour transmettre ledit premier signal numérique (SO),
- une seconde liaison parallèle (B2) reliant ladite unité (3) à l'entrée numérique de programmation dudit atténuateur (2, 2A, 2B) pour transmettre ledit second signal numérique (A)
et en ce que la sortie du convertisseur numérique-analogique (1) est reliée à l'entrée analogique de l'atténuateur (2, 2A, 2B).

6. Système de synthèse selon la revendication 5, caractérisé en ce que ladite unité de calcul est un micro-ordinateur et en ce que ladite seconde liaison parallèle (B2) comporte une carte d'interface parallèle (4) insérée entre une sortie parallèle dudit micro-ordinateur et l'entrée numérique de programmation de l'atténuateur (2, 2A, 2B).

7. Application du système de synthèse selon l'une des revendications 5 ou 6 pour effectuer des tests psycho-acoustiques, ledit signal analogique de synthèse (v) servant à activer un transducteur électro-acoustique (6), du type casque ou analogue, porté par un utilisateur.

8. Application du système de synthèse selon la revendication 7, caractérisé en ce que ledit second signal numérique (A) est modifiable par l'utilisateur.

9. Application du système de synthèse selon l'une des revendications 7 ou 8, caractérisé en ce que ledit signal de synthèse (v) peut être mélangé à un signal de bruit (b) avant d'être appliqué audit transducteur (6).

## Patentansprüche

1. Verfahren zum Erzeugen eines analogen Synthesesignals (v), ausgehend von einem digitalen Signal (S), daraus bestehend, daß:
- dem besagten digitalen Signal (S) die Form eines ersten digitalen, wellenförmigen Signals (SO), dargestellt von höchstens einer vorbestimmten Anzahl (n) von Bits (b1, b2, bn) über 1, und eines zweiten digitialen Amplitudensignals (A) gegeben wird,
- das besagte erste digitale Signal (SO) mit einem Digital-/Analogwandler (1), der für das Umwandeln von binären Ziffern mit einer höchstens mit der besagten vorbestimmten Anzahl (n) gleichliegenden Anzahl von Bits dimensioniert ist, in ein drittes analoges Signal (vO) verwandelt wird, und
- das besagte dritte analoge Signal (vO) durch das besagte zweite digitale Signal (A) anhand von mindestens einem programmierbaren Dämpfungsglied (2, 2A, 2B), das das besagte dritte analoge Signal auf seinem analogen Eingang und das besagte zweite digitale Signal (A) auf seinem digitalen Dämpfungs-Programmiereingang empfängt, moduliert wird.

2. Verfahren gemäß Patentanspruch 1, dadurch gekennzeichnet, daß die besagten ersten und zweiten digitalen Signale (SO, A) von einer Recheneinheit (3) erzeugt werden, welche mit mindestens einem, auf Wörtern von mindestens 8 Bits arbeitenden Mikroprozessor ausgestattet ist.

3. Verfahren gemäß einem der Patentansprüche 1 oder 2, dadurch gekennzeichnet, daß das besagte programmierbare Dämpfungsglied (2, 2A, 2B) so dimensioniert ist, daß es eine Dämpfung von mindestens 10 Dezibel ermöglicht.

4. Verfahren gemäß einem der Patentansprüche 1 bis 3, dadurch gekennzeichnet, daß das besagte programmierbare Dämpfungsglied (2, 2A, 2B) so ausgelegt ist, daß es eine Präzision von 5 Hundertstel Dezibel noch übersteigt.

5. System zur Synthese eines analogen Signals (v) für das Anwenden des Verfahrens gemäß einem der Patentansprüche 1 bis 4, dadurch gekennzeichnet, daß es:
- eine Recheneinheit (3) zum Erarbeiten der besagten ersten und zweiten digitalen Signale (SO, A),
- einen Digital-/Analogwandler (1),
- eine erste Parallelverbindung (B1), welche die besagte Recheneinheit (3) mit dem Eingang des besagten Digital-/Analogwandlers (1) zum Übertragen des besagten ersten digitalen Signals (SO) verbindet,
- eine zweite Parallelverbindung (B2), welche die besagte Recheneinheit (3) mit dem digitalen Programmiereingang des besagten Dämpfungsglieds (2, 2A, 2B) zum Übertragen des besagten zweiten digitalen Signals (A) verbindet,
umfaßt, sowie dadurch, daß der Ausgang des Digital-/ Analogwandlers (1) mit dem analogen Eingang des Dämpfungsglieds (2, 2A, 2B) verbunden ist.

6. Synthesesystem gemäß Patentanspruch 5, dadurch gekennzeichnet, daß die besagte Recheneinheit ein Mikrocomputer ist, und daß die besagte zweite Parallelverbindung (B2) eine parallele Schnittstellenkarte (4) umfaßt, welche zwischen einem Parallelausgang des besagten Mikrocomputers und dem digitalen Programmiereingang des Dämpfungsglieds (2, 2A, 2B) steckt.

7. Anwendung des Synthesesystems gemäß einem der Patentansprüche 5 oder 6 zum Durchführen von psychoakustischen Tests, wobei das besagte analoge Synthesesignal (v) dazu dient, einen von einem Benutzer getragenen elektroakustischen Wandler (6) vom Typ Kopfhörer oder ähnlich zu aktivieren.

8. Anwendung des Synthesesystems gemäß Patentanspruch 7, dadurch gekennzeichnet, daß das besagte zweite digitale Signal (A) vom Benutzer verändert werden kann.

9. Anwendung des Synthesesystems gemäß einem der Patentansprüche 7 oder 8, dadurch gekennzeichnet, daß das besagte Synthesesignal (v) vor dem Auflegen auf den besagten Wandler (6) mit einem Rauschsignal (b) vermischt werden kann.

## Claims

1. A process for producing an analogue signal of synthesis (v) from a digital signal (S) consisting of the following:
- putting the said digital signal S in the form of a wave-shaped digital signal SO represented at most by a predetermined number n of bits b1, b2, bn higher than 1 and then, in the form of a digital signal A of amplitude,
- converting the first said digital signal SO into a third analogue signal vO by means of a digital-to-analogue converter 1 dimensioned to convert binary numbers comprising a number of bits lower or equal to the said predetermined number n and
- modulating the said third analogue signal vO by the said second digital signal A by means of at least one programmable attenuator 2, 2A, 2B receiving the said third analogue signal and the said second digital signal A on its digital input for attenuation programming.

2. A process according to claim 1, characterised in that the said first and second digital signals (SO, A) are produced by a calculation unit 3 fitted out with at least one microprocessor working on words of at least 8 bits.

3. A process according to any of claims 1 or 2, characterised in that the said programmable attenuator 2, 2A, 2B is dimensioned to allow an attenuation of at least 10 decibels.

4. A process according to any of claims 1 to 3, characterised in that the said programmable attenuator 2, 2A, 2B is provided for reaching an accuracy higher than 5 hundredths decibels.

5. A system of synthesis of an analog signal (v) for implementing the process according to any of claims 1 to 4, characterised in that it comprises the following:
- one calculation unit 3 for working out the said first and second digital signals SO, A,
- one digital-to-analogue converter 1
- one first parallel linkage B1, connecting the said unit 3 to the input of the said digital-to-analog converter 1 to transmit the said first digital signal SO,
- a second parallel linkage B2 connecting the said unit 3 to the programming digital input of the said attenuator 2, 2A, 2B for transmitting the said second digital signal A
and in that the output of the digital-to-analog converter 1 is connected to the analog input of attenuator 2, 2A, 2B.

6. A system of synthesis according to claim 5, characterised in that the said calculation unit is a microcomputer and in that the said second parallel linkage B2 comprises a parallel interface board 4, inserted between a parallel output of the said microcomputer and the programming digital input of attenuator 2, 2A, 2B.

7. An application of the system of synthesis according to any of claims 5 or 6 for carrying out psychoacoustic tests, the said analogue signal of synthesis v being used to activate an electro-acoustic transducer 6 of the headphone type or the like, worn by the user.

8. An application of the system of synthesis according to claim 7, characterised in that the said second digital signal A may be modified by the user.

9. An application of the system of synthesis according to any of claims 7 or 8, characterised in that the said signal of synthesis v may be mixed with a signal of noise b before being applied to the said transducer 6.
